Europäisches Patentamt

European Patent Office

Office européen des brevets

(19)

(11) Veröffentlichungsnummer: **O OO4 534**
**B1**

(12)

# EUROPÄISCHE PATENTSCHRIFT

(45) Veröffentlichungstag der Patentschrift: **29.07.81**

(21) Anmeldenummer: **79100211.6**

(22) Anmeldetag: **25.01.79**

(51) Int. Cl.³: **C 07 D 233/64**

(54) Verfahren zur Herstellung von 4-Methyl-5-hydroxymethyl-imidazol.

(30) Priorität: **07.04.78 CH 3755/78**
**20.07.78 CH 3755/78**

(43) Veröffentlichungstag der Anmeldung:
**17.10.79 Patentblatt 79/21**

(45) Bekanntmachung des Hinweises auf die Patenterteilung:
**29.07.81 Patentblatt 81/30**

(84) Benannte Vertragsstaaten:
**BE DE FR GB IT NL SE**

(56) Entgegenhaltungen:
**HELVETICA CHIMICA ACTA, Vol. XXXI, 2.**
**Februar 1948, Band 7, Schweiz, H. ERLENMEYER**
**et al.: "8. Beitrag zu dem Problem der Ähnlichkeit**
**in der Chemie I", Seiten 32—40**

(73) Patentinhaber: **EPROVA Aktiengesellschaft**
**Im Laternenacker 5**
**CH-8200 Schaffhausen (CH)**

(72) Erfinder: **Müller, Hans-Rudolf, Dr. sc. techn.**
**Stettemerstrasse 24**
**CH-8207 Schaffhausen (CH)**
Erfinder: **Kündig, Werner**
**Niklausenstieg 11**
**CH-8200 Schaffhausen (CH)**
Erfinder: **Hedinger, Alfred**
**Dorfstrasse 7**
**CH-8240 Thayngen (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

### Verfahren zur Herstellung von 4-Methyl-5-hydroxymethyl-imidazol

Die vorliegende Erfindung betrifft ein Verfahren zur Herstellung von 4(5)-Methyl-5(4)-hydroxymethyl-imidazol durch Umsetzung von 4(5)-Methyl-imidazol mit Formaldehyd. Diese Herstellweise ist an sich bekannt. Bereits Windaus [Ber. d. deutschen chem. Gesellschaft 42. 759 (1909)] hat 4-Methyl-5-hydroxymethyl-imidazol durch 8-stündiges Erhitzen von 4-Methyl-imidazol mit Formaldehyd-Lösung im Rohr auf 120°C in geringen Mengen zu gewinnen vermocht. Erlenmeyer et al [Helv. Chim. Acta 31. 38 (1948)] erzielten nach diesem Verfahren bei 4—6-stündigem Erhitzen auf 120°C eine Ausbeute von 11% der Theorie. Die Hydroxymethylierung anderer Imidazole durch Umsetzung mit Formaldehyd führt nach R. Grindley et al [J. Chem. Soc. 1927. 3128—36] gewöhnlich mit Ausbeuten von etwa 40% zu den entsprechenden Hydroxymethyl-imidazolen.

Höhere Ausbeuten konnten in der Literatur nicht nachgewiesen werden.

Gemäss Staab et al [Liebigs Annalen der Chemie 715. 129 (1968)] werden die benötigten Methyl-5-hydroxymethyl-imidazole durch Reduktion von Methyl-imidazol-5-carbonsäureester besser als durch Hydroxymethylierung der Methyl-imidazole erhalten. Die gleiche Erfahrung haben auch Durant et al [J. Med. Chem. 1976. 19 (7) 925 (1976)] bei der Herstellung von 4-Methyl-5-hydroxymethyl-imidazol gemacht. Vergl. dazu auch die englische Patentschrift Nr. 1.341.376. 4-Hydroxymethylimidazol wurde auch durch elektrochemische Reduktion von Imidazol-4-carbonsäure erhalten [deutsche Offenlegungsschrift Nr. 2.538.621 (11.3.1976)].

Da die Reduktion von Carbonsäure- oder Carbonsäureester-Funktionen zur Hydroxymethyl-Gruppe stets aufwendig ist und in der Regel kostspielige Reagenzien erfordert, ist bis heute kein Verfahren bekannt, welches 4-Methyl-5-hydroxymethyl-imidazol einfach, billig und in hoher Ausbeute zu liefern vermag.

Der Erfindung lag die Aufgabe zugrunde, ein neues Verfahren zur Herstellung von 4-Methyl-5-hydroxymethyl-imidazol aufzufinden, das die oben geschilderten Nachteile der bis heute bekannten Verfahren nicht aufweist. Diese Aufgabe wurde durch die Bereitstellung des nachstehend beschriebenen Verfahrens gelöst. Es wurde gefunden, dass 4-Methyl-5-hydroxymethyl-imidazol in sehr hohen Ausbeuten und hervorragender Reinheit durch Umsetzung von 4-Methyl-imidazol mit Formaldehyd erhalten werden kann, wenn man die Umsetzung

(a) in wässerig alkalischer Lösung bei einem pH-Bereich von 11—13 und

(b) einem Temperaturbereich von 20—60°C durchführt und dazu

(c) keinen oder einen Ueberschuss von höchstens 0.5 Mol Formaldehyd pro Mol 4-Methyl-imidazol verwendet.

Die Anwesenheit eines grossen Ueberschusses an freiem Formaldehyd kann auch vermieden werden, indem man Formaldehyd im Laufe der Hydroxymethylierungs-Reaktion etwa entsprechend dem Verbrauch an diesem Reagens laufend zufügt.

Die Reaktion wird vorzugsweise in konzentrierter wässeriger Lösung und unter Anwendung von wässeriger Formalin-Lösung als Reagens durchgeführt. Anstelle von monomerem Formaldehyd kann auch polymerisiertes Formaldehyd $(CH_2O)_x$ wie z.B. Paraformaldehyd als $CH_2O$-Quelle verwendet werden. Zur Einstellung des alkalischen pH-Bereiches werden Alkalimetall- oder Erdalkalimetallhydroxyde, Alkalimetallcarbonate oder Alkalimetallsulfite verwendet. Alkalimetallhydroxyde werden bevorzugt. Der grösste Teil des gebildeten 4-Methyl-5-hydroxymethyl-imidazols (60—80% der Theorie) kann nach Neutralisation der verwendeten Base durch Zusatz einer Säure, vorzugsweise einer Mineralsäure, etwa Salz- oder Schwefelsäure und Entfernung des Wassers durch Eindampfen und durch azeotrope Entwässerung z.B. mit Isopropanol, Isobutanol oder 2-Butanol und Zusatz eines niedrigen Ketons direkt als freie Base mit einem Titer von 98—99% gewonnen werden. Im Dünnschichtchromatogramm sind keine starken Nebenflecken zu entdecken. Weitere 5—30% des Produktes lassen sich aus der Mutterlauge als Säureadditionssalz mit einer anorganischen oder organischen Säure zum beispiel als Hydrochlorid oder als 2-(4-Chlorbenzoyl)-benzoat usw. isolieren. Die Gesamtausbeute beträgt 80—90% der Theorie. Alternativ kann auch das gesamte Produkt als Säureadditions-salz z.B. als Hydrochlorid isoliert werden. Wenn man die Umsetzung mit Formaldehyd im alkalischen Milieu unter einem Schutzgas wie Stickstoff oder Argon durchführt, verfärben sich die Lösungen nicht und die erhaltenen Produkte sind farblos.

Mit diesem Verfahren wurde gegenüber dem Stande der Technik ein erheblicher Fortschritt erzielt. 4-Methyl-5-hydroxymethyl-imidazol ist damit ein wohlfeiles Produkt geworden. Es gewinnt als Zwischenprodukt für die Herstellung von Arzneimitteln zunehmende Bedeutung beispielsweise für die Herstellung von N-Cyan-N'-methyl-N''-[2 (4-methyl-5-imidazoyl)-methyl-thio -äthyl]-guanidin, das unter dem Freinamen CIMETIDINE bekannt geworden ist.

Die beanspruchten optimalen Reaktionsbedingungen sind ziemlich eng. Weicht man von ihnen ab, so sinkt die Ausbeute erheblich. Es hat sich gezeigt, dass die Einhaltung eines bestimmten hohen pH-Wertes, einer nicht zu hohen Temperatur und die Anwendung eines nur geringen Formaldehyd-Ueberschusses für die Erzielung guter Ausbeute wesentlich sind.

Einfluss von Temperatur und pH:

Im sauren oder relativ schwach alkalischen Gebiet — bis etwa pH 11 — erfolgt die Umsetzung von 4-Methyl-imidazol mit dem Formaldehyd erst bei etwas erhöhter Temperatur. Bei Raumtemperatur bilden sich unterhalb pH 11,0 keine nennenswerten Mengen von 4-Methyl-5-hydroxymethyl-imidazol. Bei pH 10,4—10,5 sind bei 30°C noch nach 65 Stunden 100% des eingesetzten 4-Methylimidazols unverändert. Mit steigendem pH steigt auch die Reaktionsgeschwindigkeit rasch an. Bei Raumtemperatur und pH 11,5 sind nach 75—100 Stunden etwa 50% des eingesetzten 4-Methyl-imidazols verbraucht. Bei pH 11,6 wurden nach 72 Stunden bei Raumtemperatur 59% 4-Methyl-5-hydroxymethyl-imidazol erhalten. Bei pH 11,5 und 30°C ist bereits nach 45 Stunden die Hälfte des eingesetzten 4-Methyl-imidazols umgesetzt. Bei pH 12,7—12,8 sind bei 30°C schon nach 10 Stunden 70% und nach 26 Stunden etwa 97% des eingesetzten 4-Methyl-imidazols hydroxymethyliert.

Gute Ausbeuten von 80—≥90% an 4-Methyl-5-hydroxymethyl-imidazol wurden bei pH 12,3—12,8 und bei Raumtemperatur nach 65—75 Stunden erzielt. Im Verlauf der Reaktion steigt der pH-Wert spontan auf 13,0—13,4 an. Der optimale Bereich für die Umsetzung liegt demnach bei pH 12—13. Durch Erwärmen wird die Reaktionsgeschwindigkeit stark erhöht und die Reaktionszeit entsprechend stark erniedrigt.

TABELLE

Umsetzung von 4-Methyl-imidazol mit $CH_2O$ bei pH 12,7 bei verschiedenen Temperaturen

| Temp. °C | Zeit in h | Umgesetztes 4-Methyl-imidazol in % des Einsatzes | Maximalausbeute an 4-Methyl-5-hydroxy-methyl-imidazol |
|---|---|---|---|
| 30 | 3 | 36 | |
| | 6 | 53 | |
| | 11 | 67 | 79 — 84 % |
| | 21 | 90 | |
| 40 | 2 | 43 | |
| | 4 | 75 | |
| | 8 | 85 | 93 % |
| | 12 | 90 | |
| | 23 | 95 | |
| 50 | 1 | 59 | |
| | 2 | 75 | |
| | 3 | 83 | 80 % |
| | 5 | 95 | |

Bei 50°C wird bereits ein verfärbtes Produkt erzeugt und die erreichbare Ausbeute ist nicht mehr optimal.

Bei noch höheren Temperaturen (90—120°C) treten stark verfärbende Nebenreaktionen in Erscheinung. Das Produkt ist noch stärker verfärbt und die Ausbeute nochmals stark reduziert.

Der optimale Temperaturbereich für die Umsetzung liegt demnach bei 30—40°C.

Formaldehyd-Ueberschuss:

Erlenmeyer et al [Helv.Chim.Acta *31*.38(1948)] setzten 2,75 Mol $CH_2O$ pro Mol 4-Methyl-imidazol ein. Seblst unter sonst optimalen Bedingungen (pH, Temperatur) werden mit diesem $CH_2O$-Ueberschuss Ausbeuten von nur 53—59% der Theorie erzielt.

Mit 1,2 Mol $CH_2O$ pro Mol 4-Methyl-imidazol stieg die Ausbeute unter sonst identischen Bedingungen auf ≥ 80%.

Mit 1—1,2 Mol $CH_2O$ wurden die höchsten Ausbeuten erzielt.

Man kann den Formaldehyd auch zudosieren oder als Formaldehyd-Quelle polymeren Formaldehyd wie z.B. Paraformaldehyd benutzen.

Einfluss des Lösungsmittels:

Am besten arbeitet man in möglichst konzentrierter wässeriger Lösung. Ersetzt man das Wasser etwa durch Methanol so bilden sich nur Spuren von 4-Methyl-5-hydroxymethyl-imidazol. Andere Lösungsmittel sind noch ungeeigneter.

Die folgenden Beispiele sollen das erfindungsgemässe Verfahren illustrieren ohne die Erfindung selbst auf diese Beispiele zu beschränken.

Beispiele:
### Beispiel 1
*4-Methyl-5-hydroxymethyl-imidazol-Hydrochlorid*

164,2 g 4-Methyl-imidazol [2,0 Mol] werden geschmolzen, mit 36 g [2 Mol] Wasser versetzt und in einer Schutsgasatmosphäre ($N_2$) unter Rühren gelöst. Nun werden 166,4 g wässerige Formalin-Lösung (36,1%ig) [2,0 Mol] unter Rühren und Kühlung derart eingetropft, dass die Temperatur nicht wesentlich über 30°C ansteigt. Das Reaktionsgemisch wird durch Zusatz von 20 ml wässeriger Natronlauge (Konzentration: 40 g NaOH pro 100 ml Lösung) auf pH 12,4 gestellt und bei 30°C gerührt.

Mittels eines pH-Reglers, der die Zugabe weiterer Natronlauge steuert, wird während der Hauptreaktionszeit das pH auf 12,2—12,4 eingestellt.

Nach 24 Stunden Reaktionszeit wird eine zweite Portion von 16,6 g Formalin-Lösung [0,2 Mol] zugefügt und die Reaktions-Lösung während weiteren 48 Stunden bei pH 12,5 gerührt.

Nach 4 Stunden seit Reaktionsbeginn war der NaOH-Verbrauch mit ca. 0,39 Mol NaOH beendet. Das pH war dann 12,2, nach 24 Stunden 12,9, nach 45 Stunden 13,1 und nach 70 Stunden 13,1.

Die Reaktionslösung wird durch Zugabe von konzentrierter Salzsäure neutralisiert, anschliessend wird das Wasser im Vakuum unter leichtem Erwärmen (Aussentemperatur 40°C) verdampft. Der Rückstand (350 g) wird in 900 ml 2-Butanol gelöst. Durch Abdestillieren von 500 ml 2-Butanol wird die Lösung azeotrop weiter entwässert.

Das ausgeschiedene Kochsalz wird durch Druckfiltration der 40°C warmen Lösung entfernt.

Das klare Filtrat wird unter Kühlung und unter Stickstoffgas bei max. 40°C mit 74 g gasförmigem Chlorwasserstoff versetzt, wobei sich das 4-Methyl-5-hydroxymethyl-imidazol in dessen Hydrochlorid umwandelt. Das Hydrochlorid kristallisiert aus. Es wird nach weiterem Einengen und nach Zusatz eines niedrigen Ketons z.B. von Aceton vollständig auskristallisieren gelassen und anschliessend genutscht.

Ausbeute: 238 g 4-Methyl-5-hydroxymethyl-imidazol-Hydrochlorid das sind 80% der Theorie.
Schmelzpunkt: ca. 240°C unter Zersetzung.
Gehalt: 100,8% der Theorie.

### Beispiel 2
*4-Methyl-5-hydroxymethyl-imidazol*

2 Mol 4-Methyl-imidazol werden ähnlich wie im Beispiel 1 beschrieben mit Formalin bei pH 12—13 und bei 40°C umgesetzt.

Bereits nach ca. 50 Stunden ist die Reaktion beendet.

Die Reaktionslösung wird mit Salzsäure neutralisiert, im Vakuum vollständig eingedampft, mit Isopropanol versetzt, azeotrop entwässert und durch Druckfiltration vom Kochsalz befreit. Das klare Filtrat wird weiter eingedampft und der Rückstand mit Aceton verrührt. Das ausgeschiedene 4-Methyl-5-hydroxymethyl-imidazol wird abgenutscht.

Ausbeute: 153 g 4-Methyl-5-hydroxymethyl-imidazol das sind 68% der Theorie.
Schmelzpunkt: 136°C

Aus der Mutterlauge lassen sich durch Einleiten von Salzsäure-Gas noch 41 g 4-Methyl-5-hydroxymethyl-imidazol-Hydrochlorid gewinnen: das sind 13,8% der Theorie. Wird die Mutterlauge statt mit Salzsäuregas mit der äquivalenten Menge 2-(4-Chlorbenzoyl)-benzoesäure versetzt, so erhält man 104 g kristallisiertes 4 - Methyl - 5 - hydroxymethyl - imidazol-[2 - (4 - chlorbenzoyl) - benzoat]: das sind 13,9% der Theorie.

Schmelzpunkt: ca. 150°C unter Zersetzung.
Äquivalentgewicht: ber. 372,8 gef. 375,0 und 373,0.

### Beispiel 3
*4-Methyl-5-hydroxymethyl-imidazol*

164,2 g 4-Methyl-imidazol [2 Mol] werden geschmolzen, mit 36 g Wasser [2 Mol] versetzt und durch Zusatz von wässeriger Natronlauge auf ein pH von 12,3 eingestellt.

Nun werden im Laufe von 24 Stunden kontinuierlich 166,4 g wässeriger Formalin-Lösung (36,1%ig) [2 Mol] in die Reaktionslösung eingepumpt. Die Temperatur wird auf 30°C gehalten. Durch einen pH- Regler der die Zugabe weiterer Mengen von Natronlauge steuert, wird das pH während der Hauptreaktionszeit auf 12,3—12,6 gehalten. Anschliessend steigt das pH — ohne weitere Zugabe von Natronlauge — allmählich auf 13,3 an. Nach beendeter Formalin-Zugabe wird noch 48 Stunden bei 30°C gerührt.

Die Reaktionslösung wird anschliessend wie im Beispiel 2 beschrieben aufgearbeitet.

Ausbeute: 176,7 g 4 - Methyl - 5 - hydroxymethyl - imidazol das sind 78,8% der Theorie.

Aus der Mutterlauge lassen sich noch 9,8 g 4 - Methyl - 5 - hydroxymethyl - imidazol·Hydrochlorid (das sind 3,3% der Theorie) gewinnen.

Gesamtausbeute: 82,1%.

Beispiel 4
*4-Methyl-5-hydroxymethyl-imidazol*
*Formaldehyd-Quelle = Paraformaldehyd*
164,2 g destilliertes 4-Methyl-imidazol (2 Mol) werden mit 54 g Wasser (3 Mol) versetzt und unter Stickstoffschutzgas unter leichtem Erwärmen gelöst.

Die Lösung wird bei 20°C portionenweise mit 66,1 g Paraformaldehyd (2,2 Mol) versetzt. Anschliessend setzt man portionenweise 10,4 g Natriumhydroxyd zu, wodurch der pH auf 12,6 steigt.

Die Reaktionslösung wird 20 Stunden bei 40°C gerührt. Der pH beträgt nun 13,1.

Aufarbeitung:
Der Kristallbrei wird mit 50 g Isopropanol versetzt, bei 40°C gerührt, unter Wasserkühlung mit 20,3 ml konzentrierter Salzsäure neutralisiert und zur Trockene eingedampft. Der Rückstand wird mit Isopropanol verrührt und im Vakuum azeotrop entwässert. Das Produkt wird in 250 ml 75°C heissem Isopropanol gelöst und in durch Filtration in der Hitze vom ausgeschiedenen Kochsalz getrennt. Das klare Filtrat wird, ähnlich wie im Beispiel 2 beschrieben, durch Eindampfen und Verrühren mit Aceton weiter aufgearbeitet. Man erhält 190 g 4-Methyl - 5 - hydroxymethyl - imidazol das sind 84.7% der Theorie.

Aus der acetonischen Mutterlauge lassen sich durch Eindampfen, Lösen des Rückstandes in Isopropanol und Versalzen mit 2 - (4 - Chlorbenzoyl) - benzoesäure noch 58 g 4-Methyl - 5 - hydroxymethyl - imidazol·[2 - (4 - chlorbenzoyl) - benzoat]; das sind 7,8% der Theorie gewinnen.

Gesamtausbeute: 92,5% der Theorie.

**Patentansprüche**

1. Verfahren zur Herstellung von 4-Methyl-5-hydroxymethyl-imidazol durch Umsetzung von 4-Methyl-imidazol mit Formaldehyd, dadurch gekennzeichnet, dass man die Umsetzung

(a) in wässerig alkalischer Lösung bei einem pH-Bereich von 11—13 und
(b) einem Temperaturbereich von 20—60°C durchführt und dazu
(c) keinen oder einen Ueberschuss von höchstens 0,5 Mol Formaldehyd pro Mol eingesetztes 4-Methyl-imidazol verwendet.

2. Verfahren nach Anspruch 1, dadurch gekennzeichnet, dass man die Umsetzung bei einem pH-Bereich von 12—13 durchführt.

3. Verfahren nach Anspruch 1, dadurch gekennzeichnet, dass man die Umsetzung bei 30—40°C durchführt.

4. Verfahren nach Anspruch 1, dadurch gekennzeichnet, dass man den Formaldehyd im Laufe der Hydroxymethylierung laufend zufügt.

5. Verfahren nach Anspruch 1, dadurch gekennzeichnet, dass man als Formaldehyd-Quelle polymeres Formaldehyd verwendet.

**Revendications**

1. Procédé de préparation de 4-méthyl-5-hydroxyméthyl-imidazole par réaction de 4-méthyl-imidazole avec du formaldéhyde, caractérisé en ce que l'on effectue la réaction:

a) dans une solution alcaline aqueuse ayant une gamme de pH de 11 à 13 et
b) dans une gamme de température de 20 à 60°C, et en ce que
c) l'on utilise pas d'excès ou un excès d'au plus 0,5 mole de formaldéhyde par mole de 4-méthyl-imidazole de départ.

2. Procédé selon la revendication 1, caractérisé en ce que l'on effectue la réaction dans une gamme de pH de 12 à 13.

3. Procédé selon la revendication 1, caractérisé en ce que l'on effectue la réaction à une température de 30 à 40°C.

4. Procédé selon la revendication 1, caractérisé en ce que l'on ajoute le formaldéhyde au cours de l'opération d'hydroxyméthylation.

5. Procédé selon la revendication 1, caractérisé en ce que l'on utilise, en tant que source de formaldéhyde, un formaldéhyde polymère.

**Claims**

1. Process for the preparation of 4-methyl-5-hydroxymethyl-imidazole by reacting 4-methyl-imidazole with formaldehyde, characterised in that the reaction is carried out

(a) in aqueous alkaline solution in a pH range of 11—13 and
(b) in a temperature range of 20—60°C, and that furthermore
(c) no excess of formaldehyde, or an excess of at most 0.5 mol of formaldehyde per mol of

4-methyl-imidazole employed is used.

2. Process according to claim 1, characterised in that the reaction is carried out in a pH range of 12—13.

3. Process according to claim 1, characterised in that the reaction is carried out at 30—40°C.

4. Process according to claim 1, characterised in that the formaldehyde is added continuously in the course of the hydroxymethylation.

5. Process according to claim 1, characterised in that polymeric formaldehyde is used as the source of formaldehyde.